# EUROPÄISCHE PATENTSCHRIFT

(11) **EP 1 933 728 B1**
(45) Veröffentlichungstag und Bekanntmachung des Hinweises auf die Patenterteilung: **12.08.2009**
(21) Anmeldenummer: 06807058.0
(22) Anmeldetag: 06.10.2006
(51) Int. Cl.: A61B 17/17, A61B 17/28

(54) **INSTRUMENT ZUM VORBEREITEN UND/ODER BEARBEITEN EINES FEMURKOPFES**
INSTRUMENT FOR PREPARING AND/OR MACHINING A FEMORAL HEAD
INSTRUMENT PERMETTANT DE PREPARER ET/OU DE TRAITER UNE TETE DE FEMUR

(30) Priorität: 06.10.2005 EP 05021794
(43) Veröffentlichungstag der Anmeldung: 25.06.2008
(73) Patentinhaber: Zimmer GmbH, 8404 Winterthur (CH)
(72) Erfinder: WILLI, Roland, CH-8413 Neftenbach (CH); HINDER, Marcel, CH-9000 St. Gallen (CH); MARCHIONE, Andreas, CH-8404 Winterthur (CH)
(86) Internationale Anmeldenummer: PCT/EP2006/067162
(87) Internationale Veröffentlichungsnummer: WO 2007/039647

(56) Entgegenhaltungen:
- EP-A- 1 477 120
- EP-A- 1 588 669
- EP-A2- 0 992 222
- DE-B- 1 164 019
- US-A- 4 896 663
- US-A- 5 817 098
- US-A- 5 951 564
- US-A1- 2005 021 042

## Beschreibung

Die Erfindung betrifft ein Instrument zum Vorbereiten und/oder Bearbeiten eines Femurkopfes.

Der Femurkopf ist an dem proximalen Ende eines Oberschenkelknochens ausgebildet und bildet zusammen mit einer Hüftgelenkpfanne eines Beckenknochens ein Hüftgelenk. Vor dem Einsetzen respektive Aufsetzen eines Implantats, welches den Femurkopf zumindest teilweise ersetzt, wird der Femurkopf mittels Instrumenten der eingangs genannten Art vorbereitet und/oder bearbeitet. Die Instrumente dienen insbesondere der Ausrichtung von Werkzeugen zur Bearbeitung des Femurkopfes am Femurkopf. Die Ausrichtung erfolgt dabei in der Regel in Bezug auf den Femurhals. Von besonderer Bedeutung ist eine auf den jeweiligen Patienten abgestimmte Femurkopfbearbeitung im Fall eines so genannten "resurfacing", bei dem nicht der gesamte natürliche Femurkopf ersetzt, sondern lediglich eine äußere Schicht an Knochenmaterial entfernt wird, um anschließend ein kappenförmiges Femurimplantat auf den verbliebenen Femurstumpf aufzusetzen, welches mit der Hüftgelenkpfanne des Beckenknochens, insbesondere einer künstlichen Hüftschale, zusammenwirkt.

Hier wird nun ein Instrument der eingangs genannten Art (siehe z.B DE-B-11 64 019) vorgeschlagen, welches, neben einer Vielzahl anderer vorteilhafter Eigenschaften, eine einfache und zugleich exakte Ausrichtung eines nachfolgend zu verwendenden Werkzeugs zur Bearbeitung eines Femurkopfes gewährleistet. Spezifischer soll es beispielsweise ermöglicht sein, eine Achse, insbesondere eine Bohrachse, für ein Werkzeug zur Bearbeitung eines Femurkopfes festzulegen. Weiterhin soll ein Verfahren zum Vorbereiten und/oder Bearbeiten eines Femurkopfes angegeben werden.

Neben anderen vorteilhaften Eigenschaften vermögen die in den Patentansprüchen beschriebenen Gegenstände auch diese Forderungen zu erfüllen.

Das angegebene Instrument gemäß Anspruch 1 umfasst eine Tastzange mit zwei an einem Drehpunkt miteinander verbundenen Zangenhebeln sowie zwei, je einem der Zangenhebel zugeordneten Tastbacken. Das Instrument weist eine Manipulierseite und eine Arbeitsseite auf, wobei die Tastbacken auf der Arbeitsseite des Instrumentes angeordnet sind. Zusätzlich ist ein Führungselement mit einer Zentrierachse vorgesehen. Das Führungselement ist an wenigstens zwei in Richtung der Zentrierachse voneinander beabstandeten Stellen derart gelagert und/oder geführt, dass die Zentrierachse unabhängig von einem Öffnungswinkel der Tastzange stets in einer Mittelebene der Tastzange liegt, wobei die Mittelebene den Drehpunkt enthält. Die Lager- und/oder Führungsstellen sind dabei direkt und/oder indirekt an der Tastzange angelenkt.

Die zangenartige Ausgestaltung des Instrumentes bietet dem Operateur einen Zugang "von oben", d.h. zumindest näherungsweise in Richtung der Femurhalsachse, indem die Tastbacken so weit geöffnet werden, dass sie an dem Femurkopf vorbei gelangen können. Ein Arbeiten "von oben" ist äußerst einfach und benötigt vor allem wenig Platz. Das anschließende Ertasten des Femurhalses erfolgt einfach durch Schließen der Tastbacken. Dabei kann der Operateur den Übergang zwischen Femurkopf und Femurhals bzw. den Halsansatz zuverlässig ertasten, indem er z.B. mit den Tastbacken gewissermaßen die Kontur des Femur im Kopfbereich "abfährt". Hierzu sind keine raumgreifenden Bewegungen des Instrumentes erforderlich, sondern das Instrument braucht hierbei lediglich relativ geringfügig geöffnet und geschlossen zu werden. Die Tastbacken können beispielsweise proximal und distal an den Femurhals angesetzt werden.

Wenn die Tastbacken am Femurhals oder am Halsansatz anliegen, ist automatisch sichergestellt, dass die Femurhalsachse sich zumindest näherungsweise in der Mittelebene des Instrumentes erstreckt, die durch den Drehpunkt der Zangenhebel und mittig zwischen den Tastbacken hindurch verläuft. Damit kann eine Mitte des Femurhalses gefunden werden, insbesondere in proximal-distaler-Richtung, und zwar unabhängig von der Breite des Femurhalses. Weiterhin ist aufgrund der Lagerung und/oder Führung des Führungselements automatisch sichergestellt, dass auch die Zentrierachse in der Mittelebene des Instruments bzw. der Tastzange liegt. Die Zentrierachse kann beispielsweise eine Bohrachse für einen Bohrdraht für ein Werkzeug zur Bearbeitung des Femurkopfes oder eine Referenzachse für eine solche Bohrachse festlegen. Dadurch, dass sich sowohl die Femurhalsachse als auch die Zentrierachse in der Mittelebene der Tastzange befinden, kann eine Ausrichtung der Zentrierachse bezüglich der Femurhalsachse erreicht werden.

Die vorstehend erläuterte Ausrichtung des Instrumentes am Femur bzw. die Ausrichtung der Zentrierachse bezüglich der Femurhalsachse wird in den meisten Fällen als eine erster Ausrichtschritt anzusehen sein, dem ggf. wenigstens ein weiterer Ausrichtschritt und/oder wenigstens eine Feinkorrektur der Lage des Instrumentes relativ zum Femurkopf bzw. der Zentrierachse relativ zu der Femurhalsachse folgen kann. Durch die Ausgestaltung des Instrumentes ist in jeder Stellung mit am Femurhals bzw. am Halsansatz anliegenden Tastbacken zumindest bezüglich der Mittelebene stets eine optimale Ausrichtung in Bezug auf die Femurhalsachse gewährleistet, wodurch die Ausrichtung der anschließend zu verwendenden Bearbeitungsinstrumente für den Operateur erheblich vereinfacht wird.

In einer Ausführungsform ist eine Funktionseinheit vorgesehen, welche eine Bohrlehre umfasst, deren Bohrachse parallel zu einer von den beiden Zangenhebeln aufgespannten Hebelebene verläuft und in der Mittelebene liegt. Insbesondere auf diese Weise kann nach erfolgter Ausrichtung des Instrumentes am Femur eine mit der Bohrachse zusammenfallende Bohrung im Femurkopf ausgebildet werden, in die dann ein draht- oder stabförmiges Führungselement eingebracht werden kann, um mit dessen Hilfe Bearbeitungswerkzeuge, insbesondere Fräser, auf zuverlässige Weise in einer hinsichtlich der Lage der Femurkappe relativ zum Femurkopf optimalen Orientierung heranzuführen und während der Bearbeitung zu positionieren und zu bewegen.

In einer Ausführungsform sind die Zangenhebel um eine feste Drehachse gegeneinander verdrehbar und mit Abstand von der Drehachse zusätzlich über wenigstens eine Gelenkhebelanordnung miteinander verbunden.

In einer weiteren Ausführungsform trägt das Führungselement, an dem die Linearführung ausgebildet ist, die Funktionseinheit und verbindet diese mit den Zangenhebeln. Die Linearführung ist beispielsweise in Form eines Langlochs vorgesehen.

In einer weiteren Ausführungsform sind die Tastbacken an verschwenkbaren Endabschnitten der Zangenhebel ausgebildet. Dabei können die verschwenkbaren Endabschnitte durch eine Gelenkhebelanordnung miteinander verbunden sein. Diese Gelenkhebelanordnung kann zusätzlich zu einer oberhalb des Gelenkbereiches gelegenen Gelenkhebelanordnung, die insbesondere eine Linearführung aufweist, vorgesehen sein.

Diese die verschwenkbaren Endabschnitte miteinander verbindende Gelenkhebelanordnung ist in einer weiteren Ausführungsform als ein Parallelogrammgelenk ausgeführt. Insbesondere hierdurch kann erreicht werden, dass beim Öffnen und Schließen des Instrumentes unabhängig von der Öffnungsweite die verschwenkbaren Endabschnitte der Zangenhebel - bzw. diejenigen Teile der verschwenkbaren Endabschnitte, die parallel zu einer von den Zangenhebeln aufgespannten Ebene liegen - stets die gleiche Orientierung relativ zueinander aufweisen, insbesondere parallel zueinander verlaufen. Hierzu ist bei optimierter Handhabung des Instrumentes dessen Platzbedarf minimiert.

Die Funktionseinheit umfasst beispielsweise wenigstens einen Peilausleger, mit dem zumindest ein Peilstab derart gekoppelt werden kann, dass sich der Peilstab parallel zur Zentrierachse erstreckt. Dabei kann ein Peilausleger verschwenkbar ausgeführt sein, und zwar um eine Achse, die parallel versetzt zur Zentrierachse verläuft oder mit dieser Achse zusammenfällt.

In einer weiteren Ausführungsform umfasst das Instrument, insbesondere die Funktionseinheit, einen Tastarm zum Abtasten des Femurhalses und/ oder des Übergangs zwischen Femurkopf und Femurhals, wobei dieser Tastarm um die Zentrierachse verschwenkbar ist. Mit einem solchen Tastarm kann ein so genanntes "Auskreiseln" durchgeführt werden, wodurch der Operateur die Ausrichtung des Instrumentes relativ zu dem Femurkopf unter Berücksichtigung zumindest eines wesentlichen Teils des gesamten Umfangs kontrollieren kann.

In einer beispielhaften Ausführungsform sind an den Tastbacken Peilstifte angebracht, deren Verbindungslinie senkrecht zur Mittelebene steht und die Zentrierachse kreuzt. Diese Peilstifte ermöglichen eine Ausmittung im Halsbereich des Femurkopfes, wenn das Instrument angesetzt wird.

In einer weiteren beispielhaften Ausführungsform ist das Instrument mit einem verstellbaren Hilfsanschlag versehen, mit dem ein zusätzlicher Bezugspunkt für den Operateur während des Ausrichtvorgangs geschaffen werden kann. Dieser Hilfsanschlag kann in der Mittelebene beispielsweise in zwei senkrecht zueinander verlaufenden Richtungen verstellbar sein.

Weitere Ausführungsformen sind in den abhängigen Ansprüchen, der Beschreibung sowie der Zeichnung angegeben.

Die verschiedenen angegebenen Ausführungsformen eines Instruments gemäß dem auf das Instrument gerichteten unabhängigen Patentanspruch beziehungsweise die dort realisierten Merkmale können selbstverständlich untereinander kombiniert werden.

Bei dem angegebenen Verfahren zum Vorbereiten und/oder Bearbeiten eines Femurkopfes, welcher an einem Femurhals ausgebildet ist, der eine Femurhalsachse aufweist, wird ein Instrument zum Vorbereiten und/oder Bearbeiten des Femurkopfes gewählt, welches eine Tastzange mit zwei Zangenhebeln und zwei Tastbacken sowie ein Führungselement mit einer Zentrierachse umfasst, welche unabhängig von einem Öffnungswinkel der Tastzange stets in einer Mittelebene der Tastzange liegt. Die Tastzange wird derart geschlossen, dass die Tastbacken symmetrisch bezüglich der Femurhalsachse auf gegenüberliegenden Seiten am Femurhals anliegen, insbesondere proximal und distal, wodurch die Zentrierachse automatisch mittig bezüglich der beiden gegenüberliegenden Seiten des Femurhalses ausgerichtet ist.

Nachfolgend wird die Erfindung anhand von in der Zeichnung illustrierten Ausführungsbeispielen näher erläutert. Dabei sollen die Ausführungsbeispiele und die Zeichnung nur instruktiv verstanden werden und sollen nicht zur Einschränkung der in den Ansprüchen beschriebenen Gegenstände dienen. Die Darstellungen in der Zeichnung sind vereinfacht; für das Verständnis der Erfindung nicht notwendige Einzelheiten sind weggelassen worden.
- Fig. 1 - 4: zeigen verschiedene Ansichten einer Ausführungsform eines Instrumentes.
- Fig. 5: zeigt das Instrument der Fig. 1 - 4 an einem zu bearbeitenden Femurkopf.
- Fig. 6 und 7: zeigen verschiedene Ansichten einer weiteren Ausführungsform eines Instrumentes.
- Fig. 8: zeigt verschiedene Ansichten einer anderen Ausführungsform eines Instrumentes.
- Fig. 9: zeigt ein Führungselement des Instrumentes von Fig. 8.
- Fig. 10 - 12: zeigen das Instrument von Fig. 8, jeweils mit einer Adaptereinrichtung.

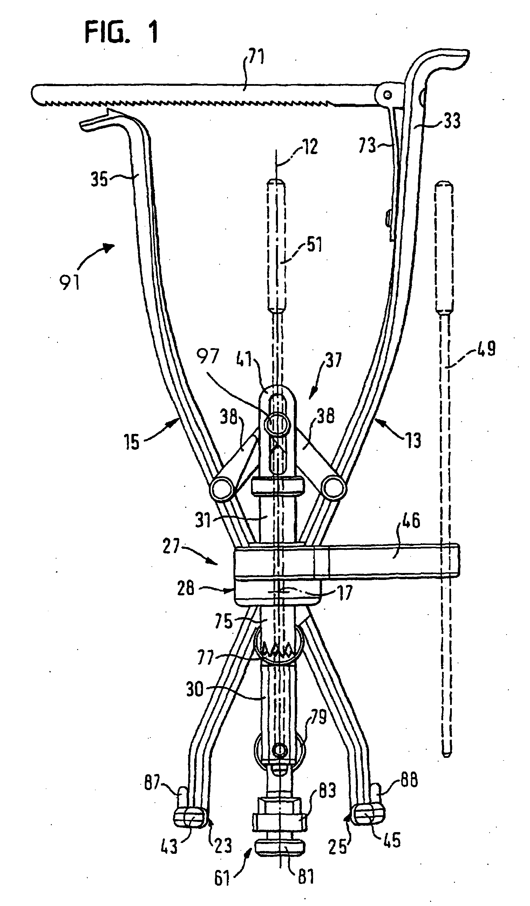

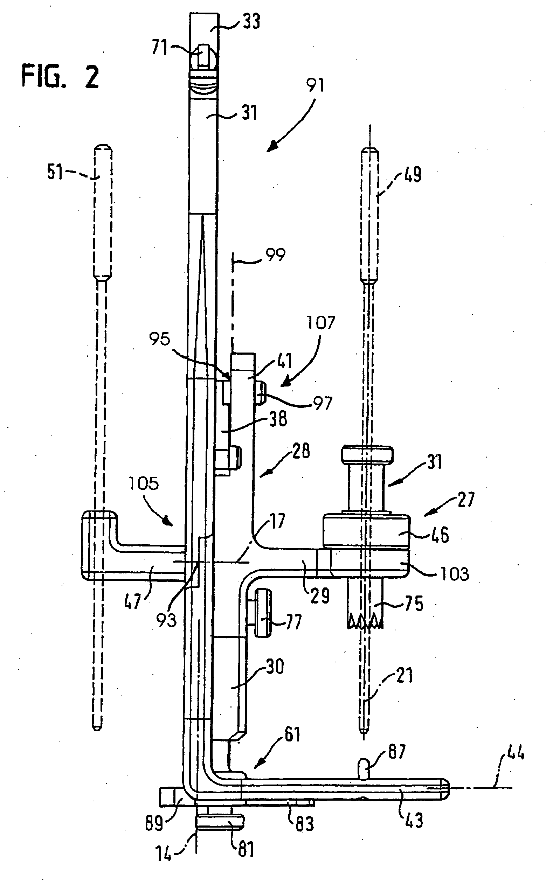

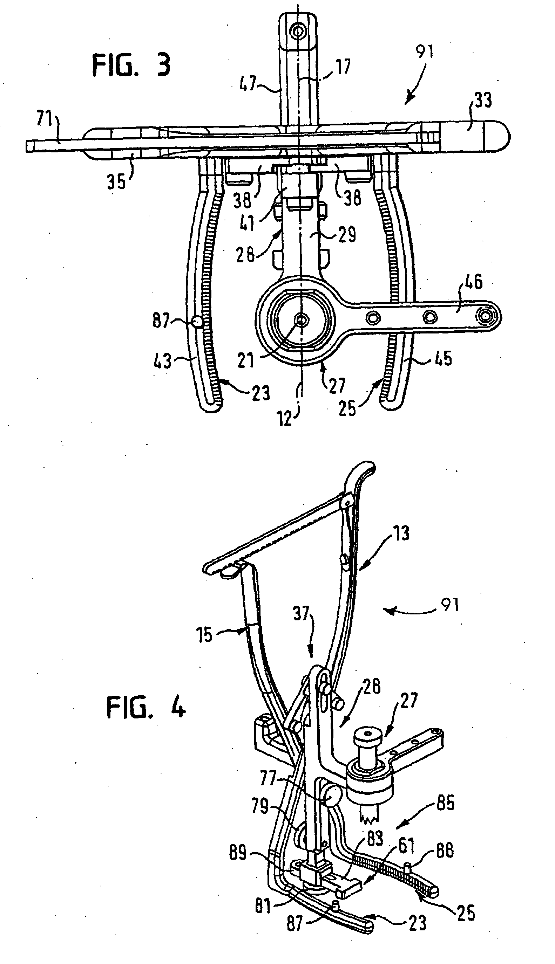

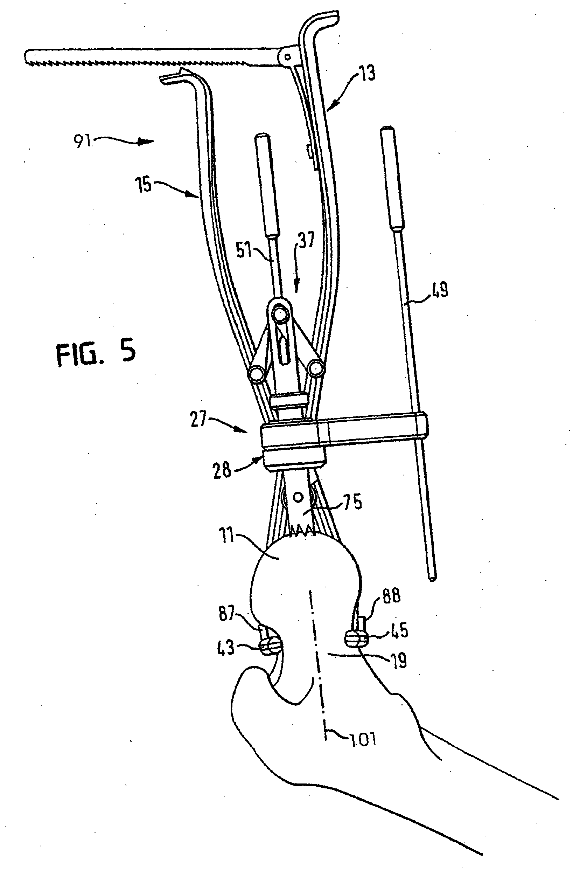

Die in den Fig. 1 - 5 dargestellte Ausführungsform eines Instrumentes umfasst eine Tastzange 91 mit zwei Zangenhebeln oder Klemmen 13, 15, die wie bei einer Schere oder Zange an einem in Fig. 2 gezeigten Drehpunkt 93, welcher auf einer Drehachse 17 der Tastzange 91 liegt, gelenkig miteinander verbunden sind, so dass die beiden Zangenhebel 13, 15 um die Drehachse 17 gegeneinander verdrehbar sind. Das Instrument ist insofern von X-förmiger Gestalt, wobei der die Drehachse 17 festlegende Gelenkbereich nicht in der Mitte der beiden Zangenhebel 13, 15 liegt, sondern diese jeweils etwa im Verhältnis von 1:2 bis 1:3 teilt. Durch die Drehachse 17 wird das Instrument in eine Manipulierseite und eine Arbeitsseite eingeteilt, wobei die Manipulierseite nachfolgend näher erläuterte Betätigungsabschnitte 33, 35 und die Arbeitsseite nachfolgend näher erläuterte Tastabschnitte 43, 45 umfasst. Einer der beiden längeren oberen Betätigungsabschnitte 33, 35 ist mit einem Rastarm 71 versehen, der an seiner zur Drehachse 17 weisenden Seite mit einer Rastverzahnung versehen ist, über die der Rastarme 71 mit dem freien Ende des anderen Betätigungsabschnitts 35 zusammenwirkt, um während einer Schließbewegung des Instrumentes die jeweils erreichte Stellung durch Verrastung zu halten. Die hierfür notwendige Vorspannung des Rastarmes 71 wird mittels einer am Betätigungsabschnitt 33 befestigten Feder 73 erreicht. Die Zangenhebel 13, 15 spannen eine Hebelebene 14 auf (Fig. 2), senkrecht zu welcher sich Tastabschnitte 43, 45 erstrecken, die an ihren einander zugewandten Innenseiten als Tastbacken 23, 25 ausgebildet sind, welche der Hebelebene 14 vorgelagert sind, wobei in Fig. 2 die Hebelebene 14 senkrecht zur Blattebene orientiert ist. Eine Mittelebene 12 der Tastzange 91 und die von den beiden Zangenhebeln 13, 15 aufgespannte Hebelebene 14 stehen senkrecht aufeinander, wobei in Fig. 1 die Mittelebene 12 senkrecht zur Blattebene orientiert ist. Ein Führungselement 28 und ein hinterer Peilausleger 47 befinden sich auf unterschiedlichen Seiten der Hebelebene 14 und sind mittels einer durch Bohrungen in den Zangenhebeln 13, 15 im Gelenkbereich hindurch verlaufenden Steckverbindung miteinander verbunden. Die Steckachse fällt mit der Drehachse 17 zusammen. Der relativ zu der Zentrierachse 21 stationäre hintere Peilausleger 47 weist ein abgewinkeltes Endstück auf, durch das ein in Fig. 1 und 2 gestrichelt angedeuteter Peilstab 51 gesteckt werden kann, der sich parallel zur Hebelebene 14 erstreckt und in einer nachstehend näher erläuterten Mittelebene 12 (Fig. 1) liegt. Das Führungselement 28 weist eine T-förmige Grundform auf, wobei der senkrechte Fuß des "T" von einem sich längs der Drehachse 17 und senkrecht zu der Hebelebene 14 erstreckenden Trägerarm 29 für die Zentrierachse 21 und für eine nachstehend näher erläuterte Funktionseinheit 27 gebildet wird. Der obere, auf der Betätigungsseite der Drehachse 17 gelegene Teil des Querbalkens des "T" ist mit einer Linearführung 41 in Form eines Langlochs versehen, das parallel zur Hebelebene 14 verläuft und in der Mittelebene 12 (Fig. 1) liegt. Die Linearführung 41 und die Tastbacken 23, 25 befinden sich auf unterschiedlichen Seiten des Gelenkbereichs. Die Mittelebene 12 verläuft durch die Drehachse 17 und mittig zwischen den beiden Zangenhebeln 13, 15 hindurch. Längs der Langlochführung 41 sind die über einen Gelenkpunkt 95 gelenkig miteinander verbundenen Enden zweier Führungshebel 38 zwangsgeführt, die mit ihren anderen Enden jeweils an die beiden Zangenhebel 13, 15 angelenkt sind. Der Gelenkpunkt 95 wird durch einen Bolzen 97 festgelegt. Folglich ist das Führungselement 28 an einer Lagerstelle 105 am Drehpunkt 93 gelagert und an einer Führungsstelle 107 am Gelenkpunkt 95 geführt. Durch diese Geometrie wird erreicht, dass die beiden Tastbacken 23, 25 der Zangenhebel 13, 15 unabhängig von der Schwenkstellung der beiden Zangenhebel 13, 15 jeweils den gleichen Abstand von der Zentrierachse 21 aufweisen. Dies bedeutet, dass die Symmetrieebene eines von den beiden Tastbacken 23, 25 gehaltenen Gegenstandes unabhängig davon, wie weit das Instrument geöffnet ist, stets mit der Zentrierachse 21 des Instrumentes zusammenfällt. Der untere, auf der Arbeitsseite der Drehachse 17 liegende Teil des Querbalkens des "T" ist im Wesentlichen von einer separaten Verlängerung 30 gebildet, die am Führungselement 28 durch eine Klemmschraube 77 gehalten ist. Ein Hilfsanschlag 61 ist an der Verlängerung 30 mittels einer weiteren Klemmschraube 79 (Fig. 4) gehalten und in der Mittelebene 12 parallel zur Hebelebene 14 verstellbar. Der Hilfsanschlag 61 weist ein separates Anschlagelement 83 auf, das wiederum in der Mittelebene 12, nunmehr jedoch senkrecht zur Hebelebene 14 und damit parallel zu einer von den Tastabschnitten 43, 45 der Zangenhebel 13, 15 aufgespannten Tastebene 44 (Fig. 2) verstellbar, wobei in Fig. 2 die Tastebene 44 senkrecht zur Blattebene orientiert ist. Das Anschlagelement 83 ist am Hilfsanschlag 61 mittels einer weiteren Klemmschraube 81 gehalten. Zwei Peilstifte 87, 88 sind jeweils auf einer Tastbacke 23, 25 angeordnet. Ihre Verbindungslinie steht senkrecht zur Mittelebene 12 und kreuzt die Zentrierachse 21. Die Tastbacken 23, 25 werden beim Schließen des Instruments so verschoben, dass sie von der Seite gesehen etwa auf der Mitte eines in Fig. 5 gezeigten Femurhalses 19 liegen. Das Anschlagelement 83 ist senkrecht zur Verbindungslinie der Peilstifte 87, 88 verschieblich einstellbar. Es besitzt eine Skala 89, welche jeweils den doppelten Abstand (zahlenmäßig) vom Hilfsanschlag 61 zur Verbindungslinie der Peilstifte 87, 88 anzeigt. Wenn in der Mittelebene 12 der Halsdurchmesser in Zahlen - z.B. durch eine vorangehende Messung mit einer Schublehre - vorliegt, dann lässt sich dieser Durchmesser in Zahlen mit der Skala 89 einstellen und der Hilfsanschlag 61 sorgt dafür, dass zumindest im Bereich der Tastbacken 23, 25 die spätere Bohrachse 21 mit der Halsachse 101 zusammenfällt. Eine vom Führungselement 28 am freien Ende des Trägerarms 29 getragene Funktionseinheit 27, welche einen vorderen Peilausleger 46 sowie eine Bohrlehre 31 umfasst, ist der Hebelebene 14 vorgelagert und über den in der Mittelebene 12 liegenden Trägerarm 29 mit den Zangenhebeln 13, 15 verbunden. Die Funktionseinheit 27 und die Tastbacken 23, 25 befinden sich auf der gleichen Seite der Hebelebene 14. Das freie Ende des Trägerarms 29 ist als kreisringförmiger Führungsbereich 103 für die Bohrlehre 31 ausgebildet. Auf dieses Ende ist mit einem ebenfalls kreisringförmigen Abschnitt der vordere Peilausleger 46 verschwenkbar und lösbar aufgesteckt, dessen abstehender Peilarm mit einer Mehrzahl von Bohrungen versehen ist, die parallel zur Hebelebene 14 und damit senkrecht zur Tastebene 44 verlaufen. Die Bohrungen dienen zur Aufnahme eines weiteren Peilstabes 49. Bei der Bohrlehre handelt es sich um eine Zentrierhülse 31, die durch die Kreisringabschnitte des vorderen Peilauslegers 46 und des Führungsbereichs 103 des Führungselements 29 hindurchgeführt ist. Die untere Stirnseite der Zentrierhülse 31 ist mit Fixierungsmitteln, einem gezahnten bzw. gezackten Rand, versehen, der einen Fixierungsabschnitt 75 bildet. Statt eines gezackten Randes können auch spitze, vorstehende Stifte vorgesehen sein. Des Weiteren weist die Zentrierhülse 31 eine zentrale Durchgangsbohrung auf, welche die Bohrachse 21 festlegt, welche in der Mittelebene 12 liegt und parallel zur Hebelebene 14 und somit senkrecht zur Tastebene 44 verläuft. Die Peilstäbe 49, 51 und ein durch die Zentrierhülse 31 hindurch gestecktes, nicht dargestelltes Bohrinstrument, beispielsweise ein Kirschnerdraht, verlaufen somit parallel zueinander. Im auf den Femur aufgesetzten Zustand (Fig. 5) liegen die Tastabschnitte 43, 45 am Femurhals 19 an. Der Femurkopf 11 befindet sich in einem Aufnahmeraum 85 des Instrumentes, der auf der einen Seite von der Tastebene 44 (Fig. 2), auf der gegenüberliegenden Seite von der Funktionseinheit 27 und senkrecht dazu von der Hebelebene 14 (Fig. 2) begrenzt ist. Damit wird von dem Instrument im Bereich des Femurkopfes 11 sehr wenig Platz benötigt. In Fig. 5 nicht dargestellt ist der Hilfsanschlag 61 (Fig. 4), dessen Anschlagelement 83 in der Tastebene 44 (Fig. 2) liegt. Das freie Ende des Anschlagelementes 83 befindet sich somit ebenfalls in Höhe des Femurhalses 19 und stellt damit eine zusätzliche Ausrichthilfe bereit. Das Instrument dient zur Bestimmung von Ort und Richtung einer im Femurkopf 11 auszubildenden Bohrung für ein insbesondere draht- oder stiftförmiges Führungselement, entlang welchem anschließend Instrumente zur Bearbeitung des Femurkopfes 11 geführt werden können. Mit dem Instrument kann der Operateur über die an den Tastabschnitten 43, 45 ausgebildeten Tastbacken 23, 25 den Übergang zwischen Femurkopf 11 und Femurhals 19 ertasten und dabei in vorteilhafter und Platz sparender Weise von oben arbeiten. Die Ausrichtung des Instrumentes am Femur kann mit Hilfe der Peilstäbe 49, 51, von denen der vordere, auf der gleichen Seite der Hebelebene 14 wie die Funktionseinheit 27 liegende Peilstab 49 um die Bohrachse 21 verschwenkt werden kann, kontrolliert werden. Von besonderem Vorteil ist, dass diese Überprüfung mit den beiden Peilstäben 49, 51 in zwei senkrecht zueinander stehenden Ebenen erfolgen kann. Mit dem Erreichen der Soll-Position kann das Instrument in dieser durch einen leichten Hammerschlag auf die Zentrierhülse 31 über den Fixierungsabschnitt 75 auf dem Femurkopf fixiert werden. Die Bedienung des Instrumentes ist für den Operateur extrem einfach und kann mit einer Hand erfolgen. Die während einer Schließbewegung erfolgende Verrastung der erreichten Schließstellung kann durch geringfügiges Anheben des Rastarmes 71 gelöst werden, um im Bedarfsfall das Instrument wieder öffnen und neu ansetzen zu können. Der Hilfsanschlag 61 sichert das Instrument gegen ein Verkippen in der Mittelebene 12. Eine grobe Voreinstellung des Hilfsanschlags 61 kann aufgrund der Operationsplanung erfolgen. Die Zwangskoppelung der betätigungsseitigen Abschnitte der Zangenhebel 13, 15 über die Führungshebel 38 mit der in der Mittelebene 12 verlaufenden Linearführung 41 sorgt automatisch für ein symmetrisches Öffnen und Schließen des Instrumentes bezüglich der Zentrierachse 21. Damit ist sichergestellt, dass die Bohrachse 21 in dieser Mittelebene 12 liegt, in der zumindest näherungsweise auch die Femurhalsachse 101 liegt, wenn die Tastbacken 23, 25 am Femurhals 19 bzw. am Halsansatz anliegen. Ist die korrekte Ausrichtung des Instrumentes am Femurkopf 11 gefunden, kann durch Schläge auf die unten gezackte oder mit spitzen Stiften versehene Zentrierhülse 31 deren Zentrierung auf dem Femurkopf 11 erfolgen, wodurch das Instrument ausreichend sicher am Femurkopf 11 fixiert ist, um durch die zentrale Bohrung der Zentrierhülse 31 hindurch die gewünschte Bohrung in den Femurkopf 11 längs der ausgerichteten Bohrachse 21 einzubringen.

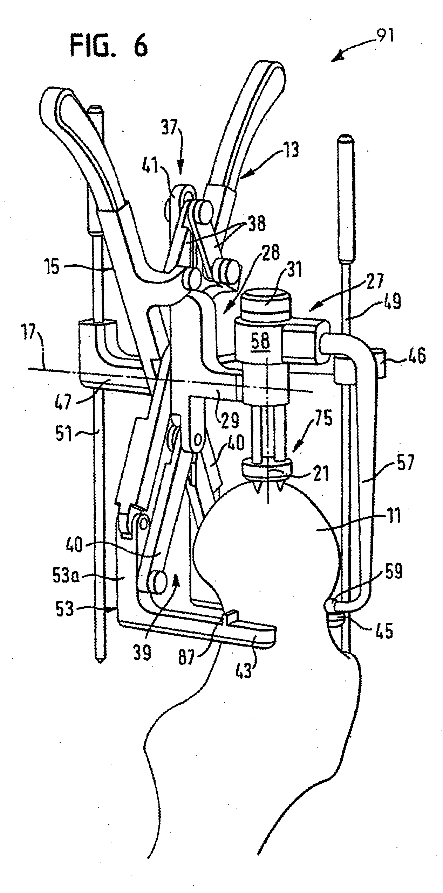

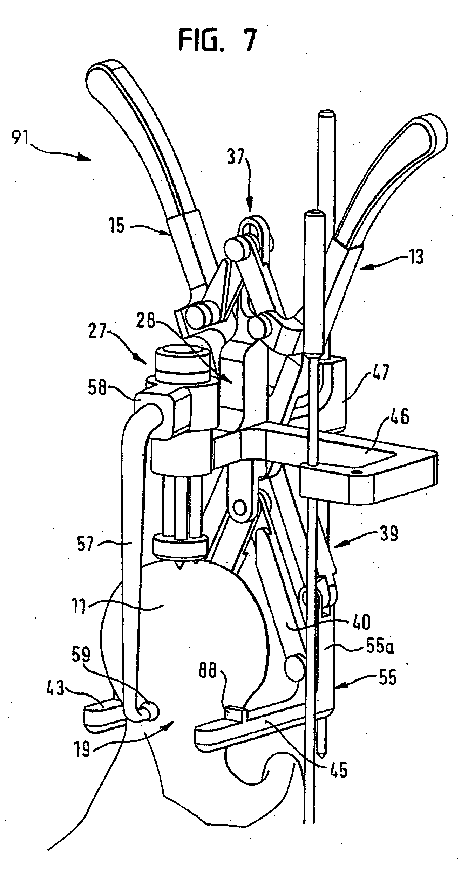

Eine weitere Ausführungsform eines Instrumentes gemäß Fig. 6 und 7 unterscheidet sich von der zuvor erläuterten Ausführungsform zunächst dadurch, dass die Tastabschnitte 43, 45 an verschwenkbaren Endabschnitten 53, 55 der Zangenhebel 13, 15 vorgesehen sind, d.h. die Zangenhebel 13, 15 sind nicht über ihre gesamte Länge starr ausgebildet, sondern mit einem Gelenk versehen. Die Schwenkachsen der Endabschnitte 53, 55 verlaufen senkrecht zur in Figur 2 dargestellten Hebelebene 14. Die Schwenkbewegung der Endabschnitte 53, 55 ist durch eine weitere, unterhalb der Drehachse 17 gelegene Gelenkanordnung 39 zwangsgesteuert, die als Parallelogrammgelenk ausgebildet ist. Zwei Führungshebel 40, die jeweils mit ihrem einen Ende an einem parallel zur Hebelebene 14 verlaufenden Teilabschnitt 53a, 55a des verschwenkbaren Endabschnitts 53, 55 angelenkt sind, sind an ihren beiden anderen Enden gemeinsam an das Führungselement 28 angelenkt. Eine in der im Zusammenhang mit Figur 1 erläuterten Mittelebene 12 liegende Verbindungsgerade zwischen dem gemeinsamen Anlenkpunkt der beiden Führungshebel 40 und der Drehachse 17 bzw. dem Drehpunkt 93 der Tastzange 91 verläuft parallel zu den Teilabschnitten 53a, 55a, wohingegen die Führungshebel 40 jeweils parallel zu demjenigen Teil des Zangenhebels 13, 15 verlaufen, der sich zwischen der Drehachse 17 und dem Anlenkpunkt des verschwenkbaren Endabschnitts 53, 55 erstreckt. Hierdurch ist auf jeder Seite der Mittelebene 12 ein Viergelenkhebel in Form eines Parallelogramms gebildet. Beim Öffnen und Schließen des Instrumentes verlaufen die beiden Teilabschnitte 53a, 55a der verschwenkbaren Endabschnitte 53, 55 somit unabhängig von der Schwenkstellung der beiden Zangenhebel 13, 15 stets parallel zueinander. Des Weiteren ist in dieser Ausführungsform der vordere Peilausleger 46 für den vorderen Peilstab 49 nicht verschwenkbar, sondern bildet einen starren Arm des Führungselements 28. Der Peilausleger 46 ist U- bzw. hakenförmig ausgebildet, liegt in einer parallel zu der im Zusammenhang mit Figur 2 erläuterten Tastebene 44 verlaufenden Ebene und ist um einen Schwenkbereich für einen nachstehend näher beschriebenen Tastarm 57 herumgeführt. Der U-förmig ausgebildete, mit seiner offenen Seite der Zentrierachse 21 zugewandte Tastarm 57 ist ein Bestandteil der Funktionseinheit 27 und mittels eines Schwenkteils 58 oberhalb der Drehachse 17 um die Bohrachse 21 herum verschwenkbar gelagert.

Der Tastarm 57 erstreckt sich rüsselartig nach unten bis kurz oberhalb der von den Tastabschnitten 43, 45 aufgespannten Tastebene 44 und dabei um den Aufnahmebereich 85 (Fig. 4) des Instrumentes für den Femurkopf 11 herum. Am freien Ende des Tastarmes 57 ist etwa in Höhe der Tastbacken 23, 25 ein kugelförmiger Tastkopf 59 ausgebildet. Durch Verschwenken des Tastarmes 57 kann das so genannte "Auskreiseln" durchgeführt werden, bei dem der Tastkopf 59 um die Bohrachse 21 herum am Halsansatz des Femurkopfes 11 entlang geführt wird, um die Lage des Instrumentes in einer parallel zur Drehachse 17 verlaufenden Richtung relativ zum Femurkopf 11 zu kontrollieren. Hierzu wird das Instrument mit den Tastabschnitten 43, 45 etwas unterhalb des Halsansatzes am Femur fixiert, damit sich der Tastkopf 59 beim Auskreiseln in Höhe des Halsansatzes bewegen kann. Ferner ist hier im Unterschied zu der vorstehend erläuterten Ausführungsform der Fixierungsabschnitt 75 nicht in Form einer "kronenartigen" Stirnseite einer Hülse vorgesehen. Vielmehr wird hier der Fixierungsabschnitt 75 von drei parallelen, unten spitz zulaufenden Stiften gebildet, die im gleichen radialen Abstand von der Bohrachse 21 und gleichmäßig in Umfangsrichtung voneinander beabstandet angeordnet sind.

Eine Auskreiseleinheit entsprechend der Ausführungsform der Fig. 6 und 7 kann auch bei der Ausführungsform gemäß Fig. 1 bis 5 vorgesehen sein. Des Weiteren kann bei beiden Ausführungsformen auch jeweils die andere Zentrierhülse 31 bzw. der jeweils andere Fixierungsabschnitt 75 vorgesehen sein. Ein Parallelogrammgelenk mit verschwenkbaren Endabschnitten entsprechend der Ausführungsform der Fig. 6 und 7 kann auch bei der Ausführungsform der Fig. 1 bis 5 vorgesehen sein.

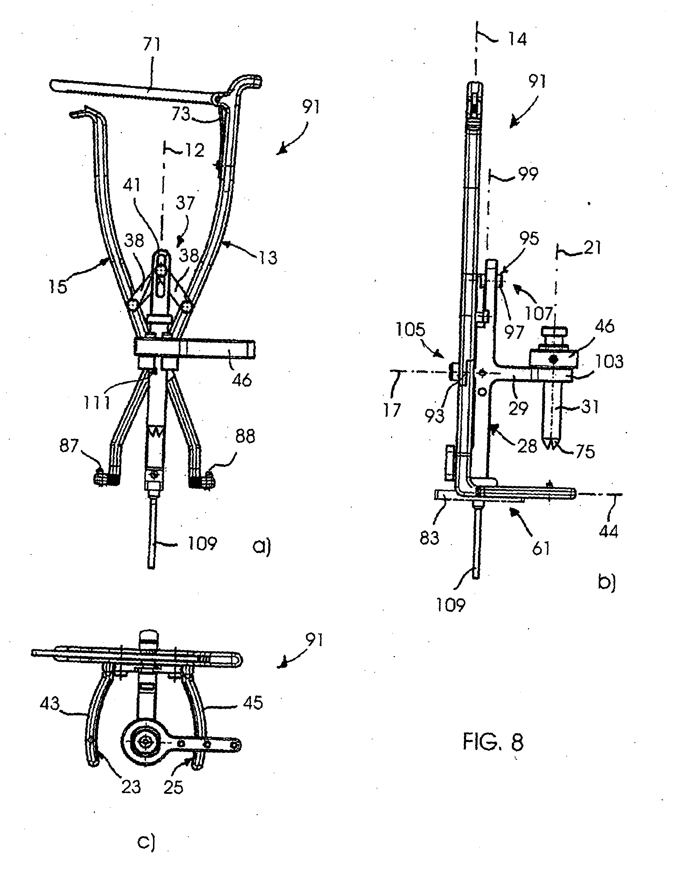

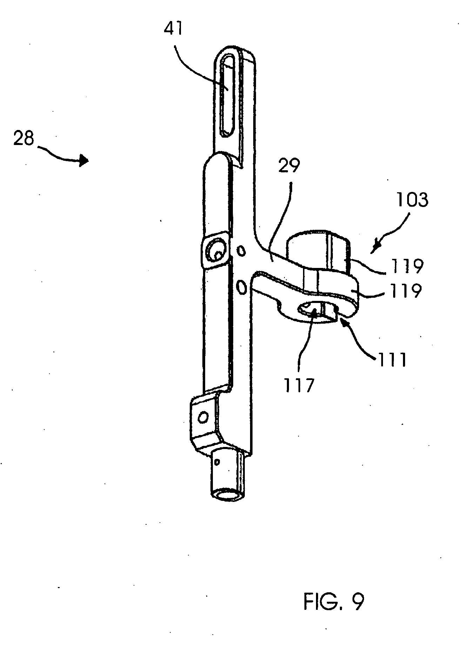

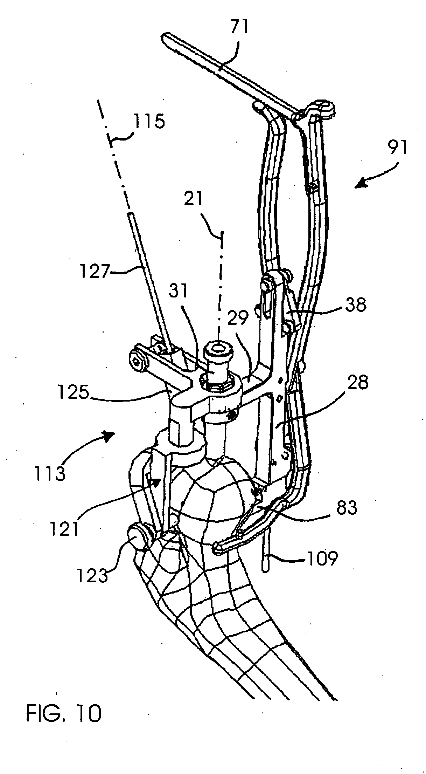

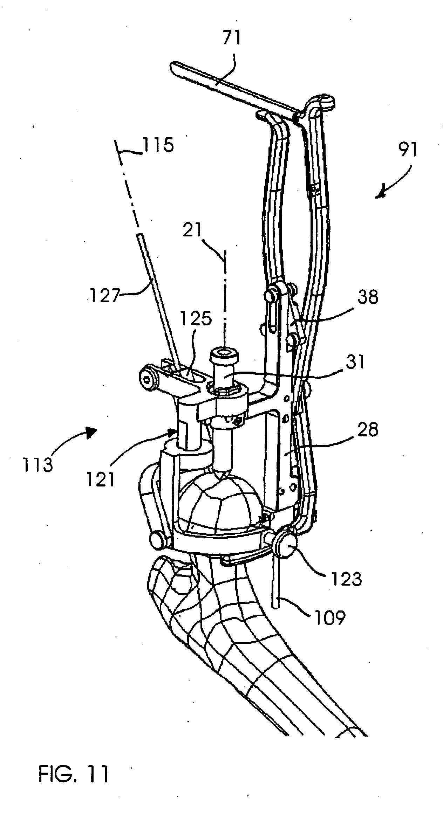

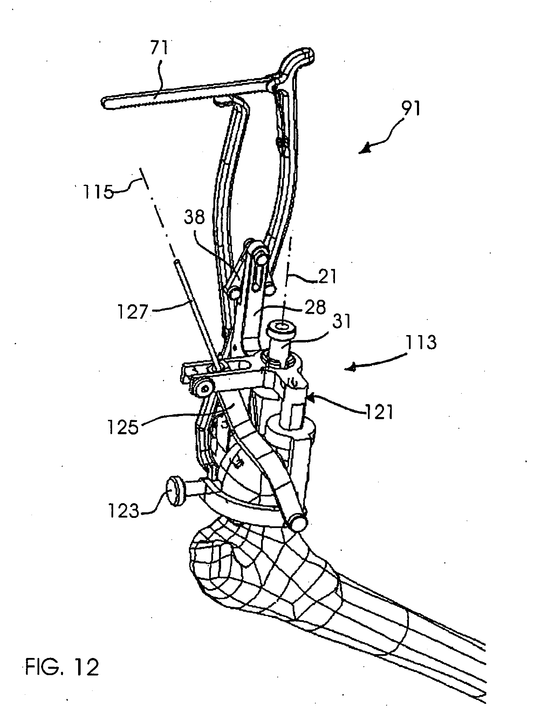

Noch eine andere Ausführungsform des Instruments gemäß Fig. 8 unterscheidet sich von den zuvor erläuterten Ausführungsformen zunächst dadurch, dass kein im Gelenkbereich der Tastzange 91 hinterer Peilausleger und kein hinterer Peilstab vorgesehen sind. Stattdessen ist in axialer Verlängerung der Führungsachse 99 ein mit dem Führungselement 28 koppelbarer unterer Peilstab 109 vorgesehen, der sich im am Führungselement 28 angebrachten Zustand über die Enden der Zangenhebel 13, 15 hinaus erstreckt. Darüber hinaus ist anstelle eines senkrecht zur Hebelebene 14 verstellbaren Anschlagelements ein Satz von Anschlagelementen 83 für den Femurhals vorgesehen, welche jeweils mit dem Führungselement 28 koppelbar sind, wobei sich das jeweils am Führungselement 28 angebrachte Anschlagelement 83 quer zur Zentrierachse 21 erstreckt und ein von der Zentrierachse entferntes freies Anschlagende aufweist. Die Anschlagelemente unterscheiden sich zumindest dadurch, dass im am Führungselement 28 angebrachten Zustand ihre freien Anschlagenden unterschiedlich weit von der Zentrierachse 21 entfernt sind. Zur Anbringung an dem Führungselement 28 wird das jeweilige Anschlagelement 83 über eine Rast- und/oder Schnappeinrichtung, insbesondere über eine Kugelverrastung, auf das Führungselements 28 aufgesteckt. Weiterhin unterscheidet sich die Ausführungsform gemäß Fig. 8 von den zuvor erläuterten Ausführungsformen durch den Führungsbereich 103. Zwar umfasst der Führungsbereich 103 der Ausführungsform gemäß Fig. 8, wie auch die zuvor erläuterten Führungsbereiche, eine Aufnahme 117 für die Zentrierhülse 31, wobei die Aufnahme 117 von einem Wandabschnitt 119 des Führungsbereichs 103 begrenzt ist, wie in Fig. 9 zu sehen ist. Allerdings ist der Führungsbereich 103 der Ausführungsform gemäß Fig. 8 geschlitzt, d.h. in dem Wandabschnitt 119 ist ein Schlitz 111 derart vorgesehen, dass der Wandabschnitt 119 die Aufnahme 117 nur teilweise umschließt. Ansonsten ist die andere Ausführungsform des Instruments gemäß Fig. 8 im Wesentlichen analog zu den zuvor erläuterten Ausführungsformen ausgebildet, wobei einige der Gemeinsamkeiten nachstehend nochmals wiederholt und/oder aus einer anderen Perspektive beleuchtet werden. So liegen auch bei dem Instrument gemäß Fig. 8 die Zentrierachse 21, die am Drehpunkt 93 angelenkte Lagerstelle 105 und die am Gelenkpunkt 95 angeordnete Führungsstelle 107 unabhängig von dem Öffnungswinkel der Tastzange 91 stets in der Mittelebene 12 der Tastzange 91. Darüber hinaus ist die durch die Lagerstelle 105 und die Führungsstelle 107 festgelegte Führungsachse 99 ebenfalls parallel zu der Zentrierachse 21 orientiert. Weiterhin ist ebenfalls ein Paar von gleichlangen Führungshebeln 38 vorgesehen, welche symmetrisch zur Mittelebene 12 angeordnet sind und die drehbar mit je einem der Zangenhebeln 13, 15 sowie an einem Gelenkpunkt 95 miteinander verbunden sind, wobei die Führungsstelle 107 an dem Gelenkpunkt 95, welcher die Führungshebel 38 miteinander verbindet, angeordnet ist. Weiterhin weist das Führungselement 28 ebenfalls ein Langloch 41 auf, wobei die Gelenkverbindung zwischen den Führungshebeln 38 durch einen Bolzen 97 gebildet ist, welcher in dem Langloch 41 des Führungselements 28 geführt ist. Weiterhin sind die Führungshebel 38 ebenfalls auf der Manipulierseite der Tastzange 91 an die Zangenhebel 13, 15 angelenkt. Weiterhin umfasst das Führungselement 28 ebenfalls einen Führungsbereich 103 für ein Zentrierelement 31, insbesondere eine Zentrierhülse, welches die Zentrierachse 21 festlegt. Weiterhin ist das Zentrierelement 31 ebenfalls in Richtung der Zentrierachse 21 verschiebbar. Weiterhin umfasst das Zentrierelement 31 an einem Ende ebenfalls Fixierungsmittel 75 zum Fixieren des Instruments am Femurkopf 11, wobei die Fixierungsmittel insbesondere zur Arbeitsseite hin angeordnet sind. Weiterhin ist der Führungsbereich 103 ebenfalls an einem sich quer zur Zentrierachse 21 erstreckenden Trägerarm 29 des Führungselements 28 ausgebildet. Weiterhin ist der Führungsbereich 103 ebenfalls mit einem Peilausleger 46 und einem dem Peilausleger 46 zugeordneten Peilstab 49 und/oder mit einer in den Fig. 10 - 12 gezeigten Adaptereinrichtung 113 zur Festlegung einer gegenüber der Zentrierachse 21 geneigten Bohrachse 115 koppelbar. Weiterhin sind ebenfalls Rastmittel 71 vorgesehen, durch welche die Zangenhebel 13, 15 in ihrer relativen Stellung zueinander verstellbar fixierbar sind. Weiterhin umfassen die Rastmittel ebenfalls einen Rastarm 71, welcher an dem einen Zangenhebel 13 angelenkt und mit dem anderen Zangenhebel 15 über eine verstellbare Rastverzahnung lösbar verrastbar ist. Die Figuren 10 bis 12 zeigen das Instrument aus Fig. 8 mit einer Adaptereinrichtung 113, durch welche eine gegenüber der Zentrierachse 21 geneigte Bohrachse 115 festlegbar ist. Die Adaptereinrichtung 113 umfasst zunächst eine Tasteinrichtung 121 mit einem Tastelement 123 zum Abtasten des Femurkopfes 11. Die Tasteinrichtung 121 ist dabei derart ausgebildet, dass das Tastelement 123 in einer Richtung parallel zur Zentrierachse 121 verstellbar ist. Hierdurch wird ermöglicht, das Tastelement 123 an verschiedenen Stellen an den Femurkopfes 11 anzusetzen. Das Tastelement 123 ist derart angeordnet, dass eine Abtastung des Femurkopfes 11 von anterior erfolgt. Darüber hinaus umfasst die Adaptereinrichtung 113 mit der Tasteinrichtung 121 gekoppelte langgestreckte Führungsmittel 125 zur Führung eines Bohrdrahtes 127 zum Erzeugen einer Bohrung im Femurkopf 11 entlang der Bohrachse 115. Die Längsachse der Führungsmittel 125 ist dabei derart orientiert, dass die Bohrachse 115, die Zentrierachse 21 und eine senkrecht zur Zentrierachse 21 verlaufende Tastebene, in der ein freies Ende des Tastelements 123 liegt, sich in einem Punkt schneiden. Die Neigung der Bohrachse 115 gegenüber der Zentrierachse 21 ist dabei stufenlos einstellbar. Durch die geneigte Bohrachse 115 wird ermöglicht, ein gegenüber der Femurhalsachse 101 verkipptes Implantat, insbesondere ein verkipptes kappenförmiges Implantat, auf den Femurkopf 11 aufzusetzen. Wie insbesondere anhand der Figuren 11 und 12 zu erkennen ist, ist nicht nur eine Tasteinrichtung 121 vorgesehen, sondern ein Satz von Tasteinrichtungen 121, wobei sich die Tasteinrichtungen 121 zumindest dadurch unterscheiden, dass die Abtastung des Femurkopfes 11 in Fig. 10 durch ein anterior angeordnetes Tastelement 123, in Fig. 11 durch ein distal angeordnetes Tastelement 123 und in Fig. 12 durch ein proximal angeordnetes Tastelement 123 erfolgt.

Nachfolgend wird ein beispielhafter Vorgang zum Festlegen der Zentrierachse 21, sowie das allfällige Festlegen der gegenüber der Zentrierachse 21 fakultativ geneigten Bohrachse 115 unter Bezugnahme auf die Figuren beschrieben. Zunächst wird der Durchmesser des Femurhalses 19 bestimmt, beispielsweise mittels eines Kalibers. Danach wird aus einem Satz von verschiedenen Anschlagelementen 83 ein an den Durchmesser des Femurhalses 19 angepasstes Anschlagelement 83 ausgewählt und an dem Instrument angebracht. Danach wird das Instrument derart an den Femurkopf bzw. Femurhals angesetzt und ausgerichtet, dass der Femurhals 19 an dem an den Durchmesser des Femurhalses 19 angepassten Anschlagelement 83 des Instruments zum Anschlag kommt, um die Zentrierachse 21 mittig bezüglich der Ansatzseite des Instruments und deren gegenüberliegenden Seite, insbesondere in anterior-posterior-Richtung, auszurichten. Anschließend wird die Tastzange 91 des Instruments derart geschlossen, dass die Tastbacken 23, 25 symmetrisch bezüglich der Femurhalsachse 101 auf gegenüberliegenden Seiten am Femurhals 19 anliegen, insbesondere proximal und distal, um die Zentrierachse 21 automatisch mittig bezüglich der beiden gegenüberliegenden Seiten des Femurhalses 19 auszurichten. Insgesamt wird hierdurch erreicht, dass nach dem Schließen der Tastzange 91 die Zentrierachse 21 mit der Femurhalsachse 101 zusammenfällt. Anschließend wird mittels des vorderen Peilstabs 49 die Inklination der Zentrierachse 21 kontrolliert und ggf. korrigiert, sowie mittels des hinteren Peilstabs 51 oder unteren Peilstabs 109 der CCD-Winkel kontrolliert und ggf. korrigiert. Danach werden die Zangenhebel 13, 15 in ihrer relative Stellung zueinander fixiert und das Instrument in der ausgerichteten Stellung am Femurkopf 11 mittels der die Zentrierachse 21 festlegenden Zentrierhülse 31 fixiert, beispielsweise durch einen leichten Hammerschlag auf das Zentrierelement 31. Soll die im Femurkopf 11 zu erzeugende Bohrung mit der Femurhalsachse 101 zusammenfallen, wird als nächstes ein Bohrdraht in den Femurkopf 11 entlang der Zentrierachse 21 durch die Zentrierhülse 31 hindurch eingesetzt. Der Bohrdraht kann in einer Ausführungsform des Verfahrens zuvor als vorderer Peilstab 49 verwendet worden sein. Danach wird der von dem Bohrdraht durchsetzte, zuvor an das Instrument gekoppelte vordere Peilausleger 46 sowie die zur Führung des Bohrdrahts dienende Zentrierhülse 31 längs des Bohrdrahts aus dem Bohrdraht ausgefädelt und entfernt. Schließlich wird das Instrument quer zur Längserstreckung des Bohrdrahts entfernt, wobei der Bohrdraht durch den am Instrument ausgebildeten Schlitz 111 hindurchgeführt wird. Der gesetzte Bohrdraht kann nun zur Orientierung eines Werkzeugs zur Bearbeitung des Femurkopfes 11 verwendet werden. Soll die im Femurkopf 11 zu erzeugende Bohrung jedoch nicht mit der Femurhalsachse 101 zusammenfallen, sondern gegenüber der Zentrierachse 21 geneigt sein, wird das Instrument mit der Adaptereinrichtung 113 gekoppelt. Danach wird der Femurkopf 11 mit der Tasteinrichtung 121 der Adaptereinrichtung 113 abgetastet, um eine zu der Zentrierachse 21 senkrecht verlaufende Äquatorialebene des Femurkopfes 11 aufzufinden. Danach wird die Neigung der Bohrachse 115 gegenüber der Zentrierachse 21 festgelegt, wobei die Bohrachse 115 derart ausgerichtet wird, dass sie durch den Schnittpunkt der Äquatorialebene mit der Zentrierachse 21 verläuft. Hierdurch wird ermöglicht, dass die Bohrachse 115 in Richtung der Kugelmitte des Femurkopfes 11 orientiert ist. Als nächstes wird die gegenüber der Zentrierachse 21 geneigte Bohrachse 115 relativ zum Instrument fixiert. Danach wird der Bohrdraht 127 in den Femurkopf 11 entlang der Bohrachse 115 eingesetzt. Schließlich wird die Adaptereinrichtung 113 und das Instrument vom Femurkopf 11 entfernt. Der gesetzte Bohrdraht 127 kann nun zur Orientierung eines Werkzeugs zur Bearbeitung des Femurkopfes 11 verwendet werden.

Das beschriebene Instrument ermöglicht insbesondere bestimmte nachfolgend beschriebene Verfahren zum Vorbereiten und/oder Bearbeiten eines Femurkopfes auszuführen.

Ein Verfahren zum Vorbereiten und/oder Bearbeiten eines Femurkopfes, welcher an einem Femurhals ausgebildet ist, der eine Femurhalsachse aufweist, umfasst, ein Instrument zum Vorbereiten und/oder Bearbeiten des Femurkopfes zu wählen, welches eine Tastzange mit zwei Zangenhebeln und zwei Tastbacken sowie ein Führungselement mit einer Zentrierachse umfasst, welche unabhängig von einem Öffnungswinkel der Tastzange stets in einer Mittelebene der Tastzange liegt, und die Tastzange derart zu schließen, dass die Tastbacken symmetrisch bezüglich der Femurhalsachse auf gegenüberliegenden Seiten am Femurhals anliegen, insbesondere proximal und distal, wodurch die Zentrierachse automatisch mittig bezüglich der beiden gegenüberliegenden Seiten des Femurhalses ausgerichtet ist.

Ein weiteres Verfahren umfasst weiter, ein Instrument zu wählen, das ein Instrument gemäß zumindest einem der Ansprüche ist.

Ein weiteres Verfahren umfasst weiter, vor dem Schließen der Tastzange das Instrument derart auszurichten, dass der Femurhals, insbesondere posterior, an einem an den Durchmesser des Femurhalses angepassten Anschlagelement des Instruments zum Anschlag kommt, so dass nach dem Schließen der Tastzange die Zentrierachse mit der Femurhalsachse zusammenfällt.

Ein weiteres Verfahren umfasst weiter, vor dem Ausrichten des Instruments ein an den Durchmesser des Femurhalses angepasstes Anschlagelement aus einem Satz von verschiedenen Anschlagelementen auszuwählen.

Ein weiteres Verfahren umfasst weiter, vor dem Auswählen des an den Durchmesser des Femurhalses angepassten Anschlagelements den Durchmesser des Femurhalses zu bestimmen.

Ein weiteres Verfahren umfasst weiter, mittels eines Peilstabs die Inklination der Zentrierachse zu kontrollieren und/oder zu korrigieren, und mittels eines weiteren Peilstabs den CCD-Winkel zu kontrollieren und/oder zu korrigieren.

Ein weiteres Verfahren umfasst weiter, die Zangenhebel in ihrer relativen Stellung zueinander zu fixieren.

Ein weiteres Verfahren umfasst weiter, das Instrument in einer ausgerichteten Stellung am Femurkopf mittels eines die Zentrierachse festlegenden Zentrierelements zu fixieren.

Ein weiteres Verfahren umfasst weiter, einen Bohrdraht in den Femurkopf entlang der Zentrierachse durch ein als Zentrierhülse ausgebildetes Zentrierelement hindurch einzusetzen.

Ein weiteres Verfahren umfasst weiter, einen von dem Bohrdraht durchsetzten, zuvor an das Instrument gekoppelten Peilausleger zur Kontrolle der Inklination der Zentrierachse längs des Bohrdrahts aus dem Bohrdraht auszufädeln und zu entfernen, und/oder die zur Führung des Bohrdrahts dienende Zentrierhülse längs des Bohrdrahts aus dem Bohrdraht auszufädeln und zu entfernen.

Ein weiteres Verfahren umfasst weiter, das Instrument quer zur Längserstreckung des Bohrdrahts zu entfernen, wobei der Bohrdraht durch einen am Instrument ausgebildeten Schlitz hindurchgeführt wird.

Ein weiteres Verfahren umfasst weiter, das Instrument mit einer Adaptereinrichtung zu koppeln, welche Mittel zur Festlegung einer gegenüber der Zentrierachse geneigten Bohrachse umfasst.

Ein weiteres Verfahren umfasst weiter, die Neigung der Bohrachse gegenüber der Zentrierachse festzulegen.

Ein weiteres Verfahren umfasst weiter, die gegenüber der Zentrierachse geneigte Bohrachse relativ zum Instrument zu fixieren.

Ein weiteres Verfahren umfasst weiter, einen Bohrdraht in den Femurkopf entlang der Bohrachse einzusetzen.

Ein weiteres Verfahren umfasst weiter, den Femurkopf mit einer Tasteinrichtung der Adaptereinrichtung abzutasten, um eine zu der Zentrierachse senkrecht verlaufende Äquatorialebene des Femurkopfes aufzufinden.

Ein weiteres Verfahren umfasst weiter, die Bohrachse derart auszurichten, dass sie durch den Schnittpunkt der Äquatorialebene mit der Zentrierachse verläuft.

Die angegebenen Merkmale der Operationsverfahren können untereinander kombiniert werden.

Im Lichte der hier gemachten Ausführungen eröffnen sich dem Fachmann weitere Ausführungsformen der in den Ansprüchen gekennzeichneten Erfindung, welche hier nicht abschließend dargestellt werden können.

### Bezugszeichenliste

- 11: Femurkopf
- 12: Mittelebene
- 13: Zangenhebel, Klemme
- 14: Hebelebene
- 15: Zangenhebel, Klemme
- 17: Drehachse
- 19: Femurhals
- 21: Zentrierachse, Bohrachse
- 23: Tastbacke
- 25: Tastbacke
- 27: Funktionseinheit
- 28: Führungselement
- 29: Trägerarm
- 30: Verlängerung
- 31: Bohrlehre, Zentrierhülse
- 33: Betätigungsabschnitt
- 35: Betätigungsabschnitt
- 37: Gelenkhebelanordnung
- 38: Führungshebel
- 39: Gelenkhebelanordnung, Parallelogrammgelenk
- 40: Führungshebel
- 41: Linearführung, Langloch
- 43: Tastabschnitt
- 44: Tastebene
- 45: Tastabschnitt
- 46: vorderer Peilausleger
- 47: hinterer Peilausleger
- 49: vorderer Peilstab
- 51: hinterer Peilstab
- 53: verschwenkbarer Endabschnitt
- 53a: Teilabschnitt
- 55: verschwenkbarer Endabschnitt
- 55a: Teilabschnitt
- 57: Tastarm
- 58: Schwenkteil
- 59: Tastkopf
- 61: Hilfsanschlag
- 71: Rastarm
- 73: Feder
- 75: Fixierungsabschnitt
- 77: Klemmschraube
- 79: Klemmschraube
- 81: Klemmschraube
- 83: Anschlagelement
- 85: Aufnahmeraum
- 87: Peilstift
- 88: Peilstift
- 89: Skala
- 91: Tastzange
- 93: Drehpunkt
- 95: Gelenkpunkt
- 97: Bolzen
- 99: Führungsachse
- 101: Femurhalsachse
- 103: Führungsbereich
- 105: Lagerstelle
- 107: Führungsstelle
- 109: unterer Peilstab
- 111: Schlitz
- 113: Adaptereinrichtung
- 115: Bohrachse
- 117: Aufnahme
- 119: Wandabschnitt
- 121: Tasteinrichtung
- 123: Tastelement
- 125: Führungsmittel
- 127: Bohrdraht

## Patentansprüche

1. Instrument zum Vorbereiten und/oder Bearbeiten eines Femurköpfes (11), welcher an einem Femurhals (19) ausgebildet ist, umfassend eine Tastzange (91), welche ihrerseits zwei Zangenhebel (13, 15) sowie zwei, je einem der Zangenhebel (13, 15) zugeordnete Tastbacken (23, 25) umfasst, und wobei das Instrument eine Manipulierseite und eine Arbeitsseite aufweist, wobei die Tastbacken auf der Arbeitsseite des Instrumentes angeordnet sind,
wobei ein Führungselement (28) mit einer Zentrierachse (21) vorgesehen ist, welches an wenigstens zwei in Richtung der Zentrierachse (21) voneinander beabstandeten Lager- und/oder Führungsstellen (105, 107) derart gelagert und/oder geführt ist, dass die Zentrierachse (21) unabhängig von einem Öffnungswinkel der Tastzange (91) stets in einer, Mittelebene (12) der Tastzange (91) liegt, wobei die Lager- und/oder Führungsstellen (105, 107) direkt und/oder indirekt an der Tastzange (91) angelenkt sind, **dadurch gekennzeichnet, dass** die Zangenhebel an einem Drehpunkt (93) miteinander verbunden sind, dass die Mittelebene (12) den Drehpunkt (93) enthält, dass wenigstens ein Paar von gleichlangen Führungshebeln (38, 40) vorgesehen ist, welche symmetrisch zur Mittelebene (12) angeordnet sind und die drehbar mit je einem der Zangenhebel (13, 15) sowie an einem Gelenkpunkt (95) miteinander verbunden sind, wobei eine der Lager- und/oder Führungsstellen (105, 107) an dem Gelenkpunkt (95) angeordnet ist, welcher die Führungshebel (38, 40) miteinander verbindet, und, dass das Führungselement (28) ein Langloch (41) aufweist, wobei die Gelenkverbindung zwischen den Führungshebeln (38, 40) durch einen Bolzen (97) gebildet ist welcher in dem Langloch (41) des Führungselements (28) geführt ist.

2. Instrument nach Anspruch 1,
**dadurch gekennzeichnet,**
**dass** die Lager- und/ oder Führungsstellen (105, 107) unabhängig von dem Öffnungswinkel der Tastzange (91) stets in der Mittelebene (12) der Tastzange (91) liegen.

3. Instrument nach Anspruch 1 oder 2,
**dadurch gekennzeichnet,**
**dass** die beiden Lager- und/oder Führungsstellen (105, 107) eine Führungsachse (99) festlegen, welche parallel zu der Zentrierachse (21) orientiert ist.

4. Instrument nach einem der vorstehenden Ansprüche,
**dadurch gekennzeichnet,**
**dass** eine der Lager- und/oder Führungsstellen (105, 107) am Drehpunkt (93) der Tastzange (91) angelenkt ist.

5. Instrument nach einem der vorstehenden,
**dadurch gekennzeichnet,**
**dass** die Führungshebel (38) auf der Manipulierseite der Tastzange (91) an die Zangenhebel (13, 15) angelenkt sind.

6. Instrument nach einem der vorstehenden Ansprüche,
**dadurch gekennzeichnet,**
**dass** das Führungselement (28) einen Führungsbereich (103) für ein Zentrierelement (31), insbesondere eine Zentrierhülse, umfasst, welches die Zentrierachse (21) festlegt.

7. Instrument nach einem der vorstehenden Ansprüche,
**dadurch gekennzeichnet,**
**dass** wenigstens ein Anschlagelement (83) für den Femurhals (19) vorgesehen ist.

8. Instrument nach einem der vorstehenden Ansprüche,
**dadurch gekennzeichnet,**
**dass** eine Adaptereinrichtung (113) vorgesehen ist, durch welche eine gegenüber der Zentrierachse (21) geneigte Bohrachse (115) festlegbar ist.

9. Instrument nach Anspruch 8,
**dadurch gekennzeichnet,**
**dass** die Neigung der Bohrachse (115) gegenüber der Zentrierachse (21) insbesondere stufenlos einstellbar ist.

10. Instrument nach einem der Ansprüche 8 oder 9,
**dadurch gekennzeichnet,**
**dass** die Adaptereinrichtung (113) Führungsmittel (125) zur Führung von Bohrmitteln (127) zum Erzeugen einer Bohrung im Femurkopf (11) entlang der Bohrachse (115) umfasst.

11. Instrument nach Anspruch 10,
**dadurch gekennzeichnet,**
**dass** die Adaptereinrichtung (113) wenigstens eine mit den Führungsmitteln (125) koppelbare Tasteinrichtung (121) mit einem Tastelement (123) zum Abtasten des Femurkopfes (11) umfasst.

12. Instrument nach Anspruch 11,
**dadurch gekennzeichnet,**
**dass** die Tasteinrichtung (121) derart ausgebildet ist, dass das Tastelement (123) in einer Richtung parallel zur Zentrierachse (21) verstellbar ist.

13. Instrument nach einem der Ansprüche 11 oder 12,
**dadurch gekennzeichnet,**
**dass** die Führungsmittel (125) derart orientiert sind, dass die Bohrachse (115), die Zentrierachse (21) und eine senkrecht zur Zentrierachse (21) verlaufende Tastebene, in der ein freies Tastende des Tastelementes (123) liegt, sich in einem Punkt schneiden.

14. Instrument nach zumindest einem der vorstehenden Ansprüche,
**dadurch gekennzeichnet,**
**dass** Rastmittel (71) vorgesehen sind, durch welche die Zangenhebel (13, 15) in ihrer relativen Stellung zueinander verstellbar fixierbar sind.

15. Instrument nach Anspruch 14,
**dadurch gekennzeichnet,**
**dass** die Rastmittel einen Rastarm (71) umfassen, welcher an dem einen Zangenhebel (13) angelenkt und mit dem anderen Zangenhebel (15) über eine verstellbare Rastverzahnung lösbar verrastbar ist.

## Claims

1. An instrument for the preparation and/or machining of a femoral head (11) which is formed at a neck (19) of a femur, including probing forceps (91) which in turn include two forcep levers (13, 15) as well as two probing jaws (23, 25) each associated with a respective one of the forcep levers (13, 15), and wherein the instrument has a manipulation side and a working side, with the probing jaws being arranged on the working side of the instrument, wherein a guide element (28) having a centering axis (21) is provided which is supported and/or guided at at least two support and/or guide points (105, 107) mutually spaced apart in the direction of the centering axis (21) such that the centering axis (21) is always disposed in a central plane (12) of the probing forceps (91) independently of an opening angle of the probing forceps (91), with the support and/or guide points (105, 107) being pivotally connected directly and/or indirectly to the probing forceps (9) 1, **characterized in that** the forcep levers are connected to one another at a fulcrum (93); **in that** the central plane (12) includes the fulcrum (93); **in that** at least one pair of guide levers (38, 40) of equal length is provided, the guide levers being arranged symmetrically to the central plane (12) and each being rotatably connected to a respective one of the forcep levers (13, 15) as well as being connected to one another at a hinge point (95), with one of the support and/or guide points (105, 107) being arranged at the hinge point (95) which connects the guide levers (38, 40) to one another; and **in that** the guide element (28) has an elongate hole (41), with the joint connection between the guide levers (38, 40) being formed by a pin (97) which is guided in the elongate hole (41) of the guide element (28).

2. An instrument in accordance with claim 1, **characterized in that** the support and/or guide points (105, 107) are always disposed in the central plane (12) of the probing forceps (91) independently of the opening angle of the probing forceps (91).

3. An instrument in accordance with claim 1 or claim 2, **characterized in that** the two support and/or guide points (105, 107) define a guide axis (99) which is oriented parallel to the centering axis (21).

4. An instrument in accordance with any one of the preceding claims, **characterized in that** one of the support and/or guide points (105, 107) is pivotally connected to the fulcrum (93) of the probing forceps (91).

5. An instrument in accordance with any one of the preceding claims, **characterized in that** the guide levers (38) are pivotally connected to the forcep levers (13, 15) on the manipulating side of the probing forceps (91).

6. An instrument in accordance with any one of the preceding claims, **characterized in that** the guide element (28) includes a guide region (103) for a centering element (31), in particular a centering sleeve which defines the centering axis (21).

7. An instrument in accordance with any one of the preceding claims, **characterized in that** at least one abutment element (83) is provided for the femoral neck (19).

8. An instrument in accordance with any one of the preceding claims, **characterized in that** an adapter device (113) is provided by which a drilling axis (115) inclined with respect to the centering axis (21) can be fixed.

9. An instrument in accordance with claim 8, **characterized in that** the inclination of the drilling axis (115) can be adjusted, in particular steplessly, with respect to the centering axis (21).

10. An instrument in accordance with one of the claims 8 or 9, **characterized in that** the adapter device (113) includes guide means (125) for the guiding of drilling means (127) for the production of a bore in the femoral head (11) along the drilling axis (115).

11. An instrument in accordance with claim 10, **characterized in that** the adapter device (113) includes at least one probing device (121) which can be coupled to the guide means (125) and has a probing element (123) for the probing of the femoral head (11).

12. An instrument in accordance with claim 11, **characterized in that** the probing device (121) is designed such that the probing element (123) is adjustable in a direction parallel to the centering axis (21).

13. An instrument in accordance with one of the claims 11 or 12, **characterized in that** the guide means (125) are oriented such that the drilling axis (115), the centering axis (21) and a probing plane extending perpendicular to the centering axis (21), in which a free probing end of the probing element (123) is disposed, intersect at a point.

14. An instrument in accordance with any one of the preceding claims, **characterized in that** latching means (71) are provided by which the forcep levers (13, 15) can be fixed adjustably in their relative positions to one another.

15. An instrument in accordance with claim 14, **characterized in that** the latching means include a latching arm (71) which is pivotally connected to one forcep lever (13) and is releasably latchable to the other forcep lever (15) via an adjustable toothed latching arrangement.

## Revendications

1. Instrument pour préparer et/ou traiter une tête de fémur (11), laquelle est réalisée sur un col de fémur (19), comprenant une pince de préhension (91) qui comprend à son tour deux leviers de pince (13, 15) ainsi que deux mâchoires de préhension (23, 25) associées chacune à l'un des leviers de pince (13, 15), dans lequel l'instrument comprend un côté manipulation et un côté travail, les mâchoires de préhension étant agencées sur le côté travail de l'instrument,
dans lequel est prévu un élément de guidage (28) avec un axe de centrage (21), lequel est monté et/ou guidé sur au moins deux emplacements de montage et/ou de guidage (105, 107) à distance l'un de l'autre en direction de l'axe de centrage (21), de telle façon que l'axe de centrage (21) se trouve toujours dans un plan médian (12) de la pince de préhension (91) indépendamment d'un angle d'ouverture de la pince de préhension (91), les emplacements de montage et/ou de guidage (105, 107) sont articulés directement et/ou indirectement sur la pince de préhension (91), **caractérisé en ce que** les leviers de pince sont reliés les uns aux autres au niveau d'un point de rotation (93), **en ce que** le plan médian (12) contient le point de rotation (93), et les emplacements de montage et/ou de guidage (105, 107) sont articulés directement et/ou indirectement sur la pince de préhension (91),
**en ce qu'**il est prévu au moins une paire de leviers de guidage (38, 40) de même longueur, lesquels sont agencés symétriquement par rapport au plan médian (12) et sont reliés en rotation à l'un des leviers de pince respectif (13, 15) et reliés les uns aux autres au niveau d'un point d'articulation (95), dans lequel l'un des emplacements de montage et/ou de guidage (105, 107) est agencé au niveau du point d'articulation (95) qui relie ensemble les leviers de guidage (38, 40), et **en ce que** l'élément de guidage (28) comprend un trou oblong (41), ladite liaison articulée entre les leviers de guidage (38, 40) est formée par un goujon (97) qui est guidé dans le trou oblong (41) de l'élément de guidage (28).

2. Instrument selon la revendication 1,
**caractérisé en ce que** les emplacements de montage et/ou de guidage (105, 107) se trouvent toujours dans le plan médian (12) de la pince de préhension (91) indépendamment de l'angle d'ouverture de la pince de préhension (91).

3. Instrument selon la revendication 1 ou 2,
**caractérisé en ce que** les deux emplacements de montage et/ou de guidage (105, 107) définissent un axe de guidage (99) qui est orienté parallèlement à l'axe de centrage (21).

4. Instrument selon l'une des revendications précédentes,
**caractérisé en ce que** l'un des emplacements de montage et/ou de guidage (105, 107) est articulé au niveau du centre de rotation (93) de la pince de préhension (91).

5. Instrument selon l'une des revendications précédentes,
**caractérisé en ce que** les leviers de guidage (38) sont articulés sur les leviers de pince (13, 15) sur le côté manipulation de la pince de préhension (91).

6. Instrument selon l'une des revendications précédentes,
**caractérisé en ce que** l'élément de guidage (28) comprend une zone de guidage (103) pour un élément de centrage (31), en particulier une douille de centrage qui définit l'axe de centrage (21).

7. Instrument selon l'une des revendications précédentes,
**caractérisé en ce qu'**il est prévu au moins un élément de butée (83) pour le col de fémur (19).

8. Instrument selon l'une des revendications précédentes,
**caractérisé en ce qu'**il est prévu un dispositif adaptateur (113), au moyen duquel peut être défini un axe de perçage (115) incliné par rapport à l'axe de centrage.

9. Instrument selon la revendication 8,
**caractérisé en ce que** l'inclinaison de l'axe de perçage (115) est réglable, en particulier en continu, par rapport à l'axe de centrage (21).

10. Instrument selon l'une des revendications 8 ou 9,
**caractérisé en ce que** le dispositif adaptateur (113) comprend des moyens de guidage (125) pour le guidage d'organes de perçage (127) pour engendrer un perçage dans la tête de fémur (11) le long de l'axe de perçage (115).

11. Instrument selon la revendication 10,
**caractérisé en ce que** le dispositif adaptateur (113) comprend au moins un dispositif de palpage (121) susceptible d'être accouplé aux organes de guidage (125), avec un élément de palpage (123) pour le palpage de la tête de fémur (11).

12. Instrument selon la revendication 11,
**caractérisé en ce que** le dispositif de palpage (121) est réalisé de telle manière que l'élément de palpage (123) est déplaçable dans une direction parallèle à l'axe de centrage (21).

13. Instrument selon l'une des revendications 11 ou 12,
**caractérisé en ce que** les moyens de guidage (125) sont orientés de telle manière que l'axe de perçage (115), l'axe de centrage (21), et un plan de palpage, perpendiculaire à l'axe de centrage (21) et dans lequel se trouve une extrémité de palpage libre de l'élément de palpage (123), se recoupent en un point.

14. Instrument selon l'une des revendications précédentes,
**caractérisé en ce qu'**il est prévu des moyens d'enclenchement (71) au moyen desquels les leviers de pince (13, 15) sont susceptibles d'être fixés de manière réglable dans leur position relative l'un par rapport à l'autre.

15. Instrument selon la revendication 14,
**caractérisé en ce que** les moyens d'enclenchement comprennent un bras d'enclenchement (71), lequel est articulé sur l'un des leviers de pince (13) et susceptible d'être enclenché de façon détachable avec l'autre levier de pince (15) via une denture d'enclenchement réglable.
